# EUROPEAN PATENT SPECIFICATION

(11) **EP 4 118 401 B1**
(45) Date of publication and mention of the grant of the patent: **29.04.2026**
(21) Application number: 21767535.4
(22) Date of filing: 11.03.2021
(51) Int. Cl.: G01F 23/26, G01F 23/22, G01F 23/02, G01F 23/263, A61M 5/168, G01F 11/02

(54) **A SENSOR FOR MEASURING CONTENTS OF A CONTAINER**
EIN SENSOR ZUR MESSUNG DES INHALTS EINES BEHÄLTERS
CAPTEUR DE MESURE DU CONTENU D'UN RÉCIPIENT

(30) Priority: 11.03.2020 US 202062988014 P
(43) Date of publication of application: 18.01.2023
(73) Proprietor: Analog Devices, Inc., Wilmington, MA 01887 (US)
(72) Inventor: DUGAS, Alan, Wilmington, Massachusetts 01887 (US); FERNANDO, Lalinda D., Wilmington, Massachusetts 01887 (US)
(74) Representative: Wallin, Nicholas James
(86) International application number: PCT/US2021/021800
(87) International publication number: WO 2021/183704

(56) References cited:
- EP-A1- 3 428 585
- EP-A1- 3 594 638
- EP-B1- 1 999 441
- EP-B1- 2 231 203
- WO-A1-2017/021226
- WO-A1-99/30117
- WO-A2-2014/166479
- JP-A- 2019 174 226
- KR-A- 20070 080 816
- US-A- 4 795 967
- US-A- 5 304 347
- US-A- 6 073 488
- US-A- 6 110 148
- US-A1- 2005 072 228
- US-A1- 2007 234 796
- US-A1- 2013 286 064
- US-B1- 6 316 864

## Description

### PRIORITY DATA

This application claims priority to U.S. provisional patent application no. 62/988,014, filed March 11, 2020 and entitled "DRUG DELIVERY MONITORING SYSTEM".

### TECHNICAL FIELD OF THE DISCLOSURE

The present invention relates to the field of capacitive sensing, in particular to a capacitive sensor for measuring contents of a container, such as a syringe used in a drug delivery system.

### BACKGROUND

In some automated drug delivery systems, a drive system automatically pushes a plunger of a syringe containing a drug to push the drug out of the syringe. Current methods for monitoring the amount of the drug that has been delivered and/or the amount of drug remaining in the syringe involve using an electro-mechanical gearing system to monitor the rotation of the drive shaft that pushes the plunger. This is an indirect measurement of the drug delivered, and it is subject to manufacturing issues and mechanical failures. For example, failures or breakdowns of the mechanical links between the motor, plunger rod, and stopper can affect measurements, as can shifts in motor rotations and gearing tolerances during manufacturing or after repeated usage of the delivery system.

Japanese patent publication JP 2019 174226 A relates to an oil quantity measuring device for measuring the quantity of an insulating oil in an oil tank that stores an insulating oil supplied to an oil-immersed insulating cable, comprising: a sensor for detecting static capacitance in a transparent tube communicating with the oil tank and filled with an insulating oil to the same oil surface level as the oil surface level of an insulating oil in the oil tank; and a measurement unit for measuring the oil surface level in the transparent tube on the basis of a change rate of the static capacitance detected by the sensor. The sensor includes a detection electrode and a grounding electrode arranged on the outside of the transparent tube so as to face each other across the transparent tube.

### BRIEF DESCRIPTION OF THE DRAWINGS

To provide a more complete understanding of the present disclosure and features and advantages thereof, reference is made to the following description, taken in conjunction with the accompanying figures, wherein like reference numerals represent like parts, in which:
FIG. 1 is a side view of a cylindrical container having a capacitive sensor, according to an example useful for understanding the invention;
FIG. 2 is a side view of a syringe having a capacitive sensor, according to an example useful for understanding the invention;
FIG. 3 is a cross-section of a portion of a container with a capacitive sensor, according to an example useful for understanding the invention;
FIG. 4 is a cross-section of a portion of a syringe with a capacitive sensor, according to an example useful for understanding the invention;
FIG. 5 is a side view of a container with rectangular electrodes, according to an example useful for understanding the invention;
FIG. 6 is a side view of a container with tapered electrodes, according to an example useful for understanding the invention;
FIG. 7 shows the container of FIG. 6 with the stopper at a different position, according to an example useful for understanding the invention;
FIG. 8 is a side view of a syringe with a plunger rod inner electrode, according to an example useful for understanding the invention;
FIG. 9 is a side view of a syringe with a tapered outer electrode and a stopper inner electrode, according to an example useful for understanding the invention;
FIG. 10 is a side view of a syringe with two tapered outer electrodes and a stopper inner electrode, according to an example useful for understanding the invention;
FIG. 11 is a perspective view of a pair of shielded electrodes, according to an example useful for understanding the invention;
FIG. 12 is a cross-section showing the shielded electrodes shown in FIG. 11, according to an example useful for understanding the invention;
FIG. 13 is a cross-section showing shielded electrodes in which the electrode shields are driven by a buffer driver, according to an example useful for understanding the invention;
FIG. 14 is a cross-section showing shielded electrodes in which the electrode shields are grounded, according to an example useful for understanding the invention;
FIG. 15 is a cross-section of a container with shielded electrodes in which the electrode shields are driven by a different voltage source from the electrodes, according to an example useful for understanding the invention;
FIG. 16 is a perspective view of a sensor electrode with inner and outer electrode shields, according to some embodiments of the present disclosure;
FIG. 17 is a cross-section of a container with the inner and outer electrode shields, according to some embodiments of the present disclosure;
FIG. 18 is a cross-section showing inner and outer electrode shields in which the inner shields are driven by a buffer driver and the outer shields are grounded, according to some embodiments of the present disclosure;
FIG. 19 is a cross-section showing inner and outer electrodes in which the inner shields are driven by a different voltage source from the electrodes and the outer shields are grounded, according to some embodiments of the present disclosure;
FIG. 20 is a block diagram showing a drug delivery system according to an example useful for understanding the invention.

### DESCRIPTION OF EXAMPLE EMBODIMENTS OF THE DISCLOSURE

### Overview

Details of one or more implementations of the subject matter described in this specification are set forth in the description below and the accompanying drawings.

A capacitive sensor for measuring contents of a container includes a pair of electrodes forming a capacitor and one or more layers of shielding. For example, a capacitive sensor can be used to directly measures the amount of drug within the syringe. Two electrodes may be positioned along the wall of the container. Alternatively, one electrode may be positioned along the wall, and another electrode positioned within the container, e.g., on the rod or stopper of a syringe. The capacitance between the two electrodes varies based on the contents of the container, e.g., the amount of drug or other substance inside the container. In a drug delivery system, the capacitance between the electrodes may vary based on the amount of drug remaining in the syringe, so the capacitance measurement directly correlates to the amount of drug remaining.

Conforming electrode shields around the container prevent or eliminate external interference from noise sources in proximity to the capacitive sensor, e.g., to femto-farad levels. In some embodiments, the electrode shields are held to a fixed potential, such as a ground. In some embodiments, a sensor system includes multiple layers of electrode shields, e.g., an inner electrode shield that has the same voltage that is applied to the measurement electrodes, and an outer electrode shield that is held to a fixed potential.

Embodiments of the present disclosure provide a sensor for measuring contents of a container, the sensor including a voltage source to generate a first voltage, a pair of electrodes coupled to the voltage source, a measurement circuit, an inner shield, and an outer shield. The pair of electrodes applies an electric field extending through at least a portion of an interior of the container. The measurement circuit measures a capacitance across the pair of electrodes. The inner shield partially encloses the pair of electrodes and has a second voltage. The outer shield partially encloses the outer shield, and the outer shield has a third voltage.

Further embodiments of the present disclosure provide a sensor for measuring the contents of a container, the sensor including a voltage source, a pair of electrodes, a measurement circuit, and a pair of electrode shields. The voltage source generates a variable voltage source. The pair of electrodes are coupled to the voltage source and apply an electric field extending through at least a portion of an interior of the container. The measurement circuit measures a capacitance across the pair of electrodes. The pair of electrode shields partially encloses the pair of electrodes, and the pair of electrode shields are set to a fixed voltage potential.

Additional examples useful for understanding the invention provide a drug delivery system that includes a syringe holder, a stopper actuator, and a sensor. The syringe holder holds a syringe containing a drug for delivery to a patient. The stopper actuator is couplable to the syringe and controls delivery of the drug to the patient. The sensor includes a voltage source to generate a first voltage, the first voltage applied to a pair of electrodes to apply an electric field extending through at least a portion of the syringe; a measurement circuit to measure a capacitance across the pair of electrodes; and a processor to generate an instruction to the stopper actuator to deliver the drug to the patient, the instruction based on the measured capacitance.

As will be appreciated by one skilled in the art, aspects of the present disclosure, in particular aspects of a capacitive sensor for measuring contents of a syringe or other container, described herein, may be embodied in various manners (e.g., as a method, a system, a computer program product, or a computer-readable storage medium). Accordingly, aspects of the present disclosure may take the form of a hardware embodiment, a software embodiment (including firmware, resident software, micro-code, etc.), or an embodiment combining software and hardware aspects that may all generally be referred to herein as a "circuit," "module" or "system." Functions described in this disclosure may be implemented as an algorithm executed by one or more hardware processing units, e.g. one or more microprocessors, of one or more computers. In various embodiments, different steps and portions of the steps of each of the methods described herein may be performed by different processing units. Furthermore, aspects of the present disclosure may take the form of a computer program product embodied in one or more computer-readable medium(s), preferably non-transitory, having computer-readable program code embodied, e.g., stored, thereon. In various embodiments, such a computer program may, for example, be downloaded (updated) to the existing devices and systems (e.g. to the existing perception system devices and/or their controllers, etc.) or be stored upon manufacturing of these devices and systems.

The following detailed description presents various descriptions of specific certain embodiments. In the following description, reference is made to the drawings where like reference numerals can indicate identical or functionally similar elements. It will be understood that elements illustrated in the drawings are not necessarily drawn to scale. Moreover, it will be understood that certain embodiments can include more elements than illustrated in a drawing and/or a subset of the elements illustrated in a drawing.

The following disclosure describes various illustrative embodiments and examples for implementing the features and functionality of the present disclosure. While particular components, arrangements, and/or features are described below in connection with various example embodiments, these are merely examples used to simplify the present disclosure and are not intended to be limiting. It will of course be appreciated that in the development of any actual embodiment, numerous implementation-specific decisions must be made to achieve the developer's specific goals, including compliance with system, business, and/or legal constraints, which may vary from one implementation to another. Moreover, it will be appreciated that, while such a development effort might be complex and time-consuming; it would nevertheless be a routine undertaking for those of ordinary skill in the art having the benefit of this disclosure.

In the Specification, reference may be made to the spatial relationships between various components and to the spatial orientation of various aspects of components as depicted in the attached drawings. However, as will be recognized by those skilled in the art after a complete reading of the present disclosure, the devices, components, members, apparatuses, etc. described herein may be positioned in any desired orientation. Thus, the use of terms such as "above", "below", "upper", "lower", "top", "bottom", or other similar terms to describe a spatial relationship between various components or to describe the spatial orientation of aspects of such components, should be understood to describe a relative relationship between the components or a spatial orientation of aspects of such components, respectively, as the components described herein may be oriented in any desired direction. When used to describe a range of dimensions or other characteristics (e.g., time, pressure, temperature, length, width, etc.) of an element, operations, and/or conditions, the phrase "between X and Y" represents a range that includes X and Y.

Other features and advantages of the disclosure will be apparent from the following description and the claims.

### Capacitive Sensor Overview

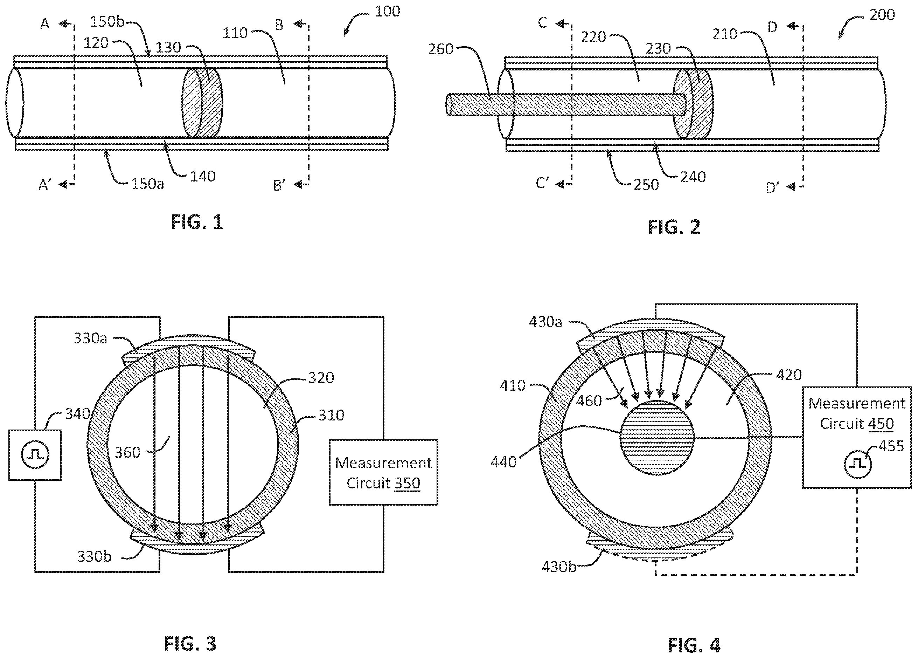

FIG. 1 is a side view of a cylindrical container having a capacitive sensor, according to an example useful for understanding the invention. The container 100 has two interior chambers 110 and 120, which are separated from each other by a stopper 130. For example, the container 100 is a syringe, and one of the chambers 110 holds a drug or other substance for delivery to a patient. The other chamber 120 is an air chamber. In the orientation shown in FIG. 1, the stopper 130 moves towards the right to eject the contents of the chamber 110, e.g., through a needle (not shown) attached to the syringe. The stopper 130 may be connected to a plunger rod, as shown in FIG. 2, that controls the position of the stopper 130. Alternatively, the position of the stopper 130 may be controlled by air pressure in the air chamber 120 or another mechanism.

The chambers 110 and 120 are enclosed by a wall 140, which may be glass, plastic, or another material. An inner side of the wall 140, referred to as an inner wall, is in contact with the chambers 110 and 120 and the stopper 130. A pair of electrodes 150 extend along an outer side of the wall 140, referred to as an outer wall. Alternatively, the electrodes 150 may extend along an inner side of the wall 140, referred to as the inner wall, or the electrodes 150 may be arranged within the walls 140 of the container. The electrodes 150 are on opposite sides of the wall 140; in the orientation shown in FIG. 1, one electrode 150a is along the bottom of the container 100, and the other electrode 150b is along the top of the container 100. In alternate embodiments, the container 100 includes a single chamber and no stopper 130 (e.g., the container 100 is a vial for holding a substance).

The electrodes 150 create an electric field within the container 100. The contents of the container 100 (i.e., the contents of the chambers 110 and 120 and the stopper 130) and the wall 140 are dielectric materials between the electrodes 150. These dielectric materials and the electrodes 150 form a capacitor. Capacitance varies based on the permittivity of dielectric material between the electrodes. If the stopper 130 moves left to right through the container 100, e.g., to push a drug out of a syringe, the volume of material in the chamber 110 decreases while the volume of material in the chamber 120 increases. In particular, if the stopper 130 pushes a drug out of a syringe, the amount of drug between the electrodes 150 decreases, and its volume is replaced with air in the other chamber 120. If the contents of the two chambers 110 and 120 have different permittivities, the measured capacitance corresponds to a change in the relative volumes of the two chambers 110 and 120. Typically drugs have a higher permittivity than air, so the measured capacitance decreases as the drug is pushed out of the container 100 and the volume of air between the electrodes 150 increases.

Two cross-sections, A-A' and B-B', are noted in FIG. 1. These two cross-sections, located on different sides of the stopper 130, may be similar but for the different contents of the chambers. In particular, the two cross-sections A-A' and B-B' have the same structure, but during use of the container 100, the two chambers 110 and 120 may be filled with different materials having different permittivities, e.g., chamber 110 is filled with air and chamber 120 is filled with a drug. FIG. 3, discussed below, represents an example of the cross-sections A-A' and B-B'.

FIG. 2 is a side view of a syringe 200 having a capacitive sensor, according to an example useful for understanding the invention. The syringe 200 includes a chamber 210, similar to the chamber 110 in FIG. 1, for holding a drug or other substance. The syringe 200 includes an air chamber 220, similar to the chamber 120 in FIG. 1. A stopper 230, similar to the stopper 130 in FIG. 1, separates the two chambers 210 and 220. A plunger rod 260 is coupled to the stopper 230 to control the position of the stopper 230. A needle or other outlet (not shown in FIG. 2) may be on the right side of the chamber 210, opposite the stopper 230. When the plunger rod 260 pushes the stopper 230 towards the right, the stopper 230 expels material from the chamber 210 and through the needle or other outlet.

The syringe 200 further includes a wall 240, which is similar to the wall 140, and at least one electrode 250 along an outer side of the wall 240. In some embodiments, the syringe 200 includes two electrodes 250 along the outer wall, similar to the electrodes 150a and 150b shown in FIG. 1. As noted with respect to FIG. 1, in other embodiments the electrodes 250 may extend along an inner side of the wall 240, or the electrodes 250 may be arranged within the walls 240 of the container. In some embodiments, the stopper 230 and/or plunger rod 260 includes an electrode, e.g., the plunger rod 260 is a conductive material that forms an electrode, or the stopper 230 is wrapped by a conductive material that forms an electrode. A plunger rod or stopper electrode is referred to as an inner electrode, e.g., an electrode inside the syringe 200, and the electrode 250 is referred to as an outer electrode, e.g., an electrode external to the syringe 200. In some embodiments, the syringe 200 includes two outer electrodes and an inner electrode.

Two cross-sections, C-C' and D-D', are noted in FIG. 2. An example of the cross-section C-C', which includes the plunger rod 260, is shown in FIG. 4, discussed below. An example of the cross-section D-D', which does not include the plunger rod 260, is shown in FIG. 3.

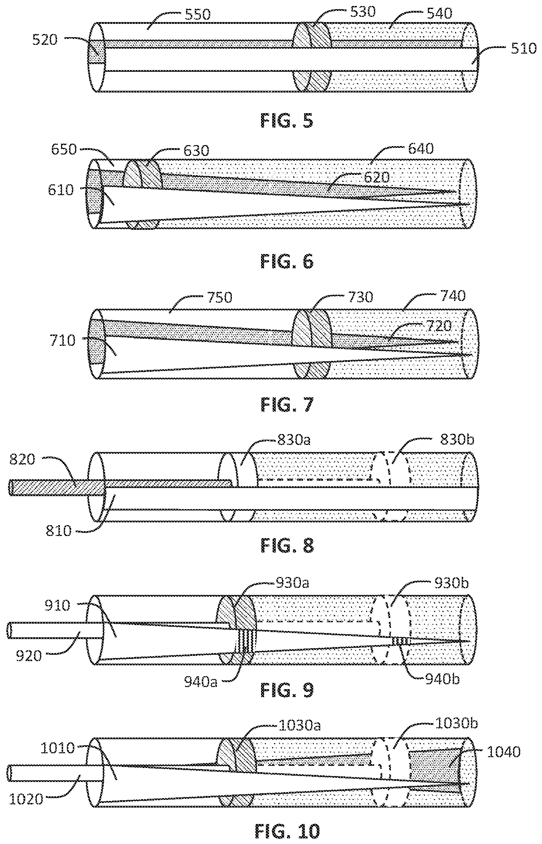

FIG. 3 is a cross-section of a portion of a container with a capacitive sensor, according to an example useful for understanding the invention. The cross-section includes a container wall 310, which may correspond to the wall 140 or 240. The wall 310 surrounds a chamber 320, which may correspond to any of the chambers 110, 120, or 210 discussed above. On the outside of the wall 310 are two electrodes 330a and 330b. The electrodes 330a and 330b are positioned on opposite sides of the wall 310. The electrodes 330a and 330b may be physically attached to the wall 310. For example, the electrodes 330a and 330b may be metal strips that are pasted or 3D printed to the wall 310. The electrodes 330a and 330b may have electrical connections configured to connect to a drug delivery monitoring system, e.g., wires or conductive patches. Alternatively, the electrodes 330a and 330b may be incorporated into a drug delivery device that can hold and dispense drugs from the syringe 200, or other another type of device for holding the container 100. When the container 100 or syringe 200 is loaded into the device, the electrodes 330a and 330b are positioned relative to the container 100 or syringe 200 as illustrated in FIGs. 1-4. In the embodiment shown in FIG. 2, if the plunger rod 260 and/or stopper 230 include an electrode, one of the electrodes 330a or 330b may be removed and only one electrode 330 is along the outer wall; this embodiment is described further with respect to FIGs. 4 and 9.

The electrodes 330a and 330b are electrically coupled to a voltage source 340. The voltage source 340 generates a voltage signal that is applied across the electrodes 330a and 330b. The voltage signal creates an electric field 360 between the electrodes 330a and 330b. The electric field 360 extends across at least a portion of the chamber 320. In this example, the voltage source 340 is a square wave source that generates a square wave voltage signal that is applied across the electrodes 330a and 330b. In other examples, other voltage stimulus signals, such as a sinusoidal or triangle voltage wave, may be used. The voltage signal may be a periodic signal with a fixed amplitude and frequency. Alternatively, the amplitude and/or frequency of the voltage signal may vary, e.g., based on instructions from a processor connected to the voltage source 340. While the electric field 360 is depicted by electric field lines that span from electrode 330a to 330b, it should be understood that the direction of the electric field may change based on the voltage signal applied by the voltage source 340.

The electrodes 330a and 330b are also electrically coupled to a measurement circuit 350. The measurement circuit 350 measures a capacitance across the electrodes 330a and 330b. Different contents of the chamber 320 have different permittivities, which affect the electric field 360 between the electrodes 330a and 330b. The capacitance measurement captured by the measurement circuit 350 reflects the contents of the chamber 320. In some embodiments, the measurement circuit 350 and voltage source 340 are incorporated on a single chip or device.

FIG. 4 is a cross-section of a portion of a syringe with a capacitive sensor, according to an example useful for understanding the invention. The cross-section includes a container wall 410, which corresponds to the wall 240. The wall 410 surrounds a chamber 420, which corresponds to the air chamber 220 in FIG. 2. On the outside of the wall 410 is a first outer electrode 430a. In some embodiments, a second outer electrode 430b (shown with a dashed line) may be arranged on an opposite side of the wall 410 from the first outer electrode 430a. The electrodes 430a and 430b are similar to the electrodes 330a and 330b described with respect to FIG. 3. A plunger rod 440 within the chamber 420 corresponds to the plunger rod 260 shown in FIG. 2. The plunger rod 440 is an inner electrode that can form a capacitor with the first outer electrode 430a. The plunger rod 440 is located within the inner side of the wall 410. In embodiments with the second outer electrode 430b, the plunger rod 440 can also form a capacitor with the second outer electrode 430b. For example, a voltage source 455 can apply a voltage difference between the plunger rod 440 and the first outer electrode 430a, or between the plunger rod 440 and the second outer electrode 430b.

The outer electrodes 430a and 430b and plunger rod 440 are electrically coupled to a measurement circuit 450, which in this example includes a voltage source 455. The voltage source 455 is similar to the voltage source 340, and the measurement circuit 450 is similar to the measurement circuit 350. In this example, an electric field 460 extends between the first outer electrode 430a and the plunger rod 440. The electric field 460 extends across a portion of the chamber 420, as illustrated in FIG. 4.

### Example Electrode Configurations

FIG. 5 is a side view of a container with rectangular electrodes, according to an example useful for understanding the invention. In this example, two electrodes 510 and 520 are arranged on opposite sides of the container. The electrodes 510 and 520 have rectangular shapes, and are curved to conform to the cylindrical container. If the container holds different materials on different sides of the stopper 530 (e.g., a drug 540 on one side and air 550 on the other side), as the stopper 530 moves through the container, the measured capacitance between the two electrodes 510 and 520 varies linearly with position. Thus, the measured capacitance indicates the amount of drug remaining in the container.

FIG. 6 is a side view of a container with tapered electrodes, according to an example useful for understanding the invention. Relative to rectangular electrodes shown in FIG. 5, tapered electrodes that taper toward the discharge end of the container lead to a sharper drop in capacitance as the material in the container is pushed out. In FIG. 6, two electrodes 610 and 620 are arranged on opposite sides of the container. The electrodes 610 and 620 are each tapered, having a narrow end at the right side and a wider end at the left side. In this example, the electrodes 610 and 620 are triangular, but in other examples, the electrodes 610 and 620 may be trapezoidal or have another tapered shape.

The capacitance between the electrodes 610 and 620 varies based on the material between the electrodes and the overlapping area of the electrodes 610 and 620. In particular, capacitance is proportional to both the overlapping area between the electrodes and the relative permittivity of the dielectric between the electrodes. The electrodes 610 and 620 are at their widest at the left side of the container in the orientation shown in FIG. 6. This portion of the electrodes 610 and 620 has the largest overlapping area. By contrast, the right side of the container has the narrowest portion of the electrodes 610 and 620, and this portion has the smallest overlapping area. At the position of the stopper 630 shown in FIG. 6, the capacitance measurement is relatively high, since the portion of the container having the higher-permittivity drug 640 extends nearly to the highest-overlap portion of the electrodes 610 and 620. The portion of the container holding the air 650 contributes a relatively small amount of capacitance to the overall capacitance between the electrodes 610 and 620.

FIG. 7 shows the container of FIG. 6 with the stopper at a different position, according to an example useful for understanding the invention. The electrodes 710 and 720 correspond to the electrodes 610 and 620, and the stopper 730 corresponds to the stopper 630. The stopper 730 has been pushed to the right in the orientation shown in FIG. 7, such that the volume of the drug 740 in the container is less than the volume of the drug 640 in FIG. 6, and the volume of air 750 in the container is greater than the volume of the air 650 in FIG. 6. In this example, the volume of the drug 740 in FIG. 7 is half of the volume of the drug 640 in FIG. 6. The capacitance for the portion of the electrodes 710 and 720 surrounding the drug 740 is less than half of the capacitance for the portion of the electrodes 610 and 620 surrounding the drug 640. This is because the area of the electrodes 710 and 720 surrounding the drug 740 is relatively small due to their tapered shape; the area of the electrodes 610 and 620 surrounding the drug 640 is more than twice the area of the electrodes 710 and 720 surrounding the drug 740.

Furthermore, while the position of the stopper 730 is the same as the position of the stopper 530 in FIG. 5, the overall capacitance measurement across the electrodes 710 and 720 is less than the overall capacitance across the electrodes 510 and 520. If the stoppers 530 and 730 were all the way to the left in their containers, e.g., the containers in FIGs. 5 and 7 were full of the drug 540 and 740, the capacitance measurements for the containers are the same if the overall areas of the electrodes 510, 520, 710, and 720 are equal. Thus, the electrode arrangement shown in FIGs. 6 and 7 results in a steeper capacitance curve relative to the arrangement shown in FIG. 5. This may improve the precision of a volume measurement based on measured capacitance.

FIG. 8 is a side view of a syringe with a plunger rod inner electrode, according to an example useful for understanding the invention. In this example, an electrode 810 is arranged on an outer wall of the container. The electrode 810 has a rectangular shape, but in other embodiments, the electrode 810 may be tapered or have another shape. A plunger rod 820 is a second electrode. The plunger rod 820 is connected to a stopper 830 at a first position 830a. The plunger rod 820 moves through the container to push the stopper 830, e.g., to eject a drug or other material from the container. A second example stopper position 830b is shown in FIG. 8. In this example, the wall of the syringe and air in the air chamber form the dielectric between the two electrodes 810 and 820; the drug or other substance held by the syringe is not between the electrodes 810 and 820 and capacitance through the drug is not measured.

As the plunger rod 820 and stopper 830 move within the container, the amount of overlap between the plunger rod 820 and outer electrode 810 increases. For example, at the stopper position 830b, the area of overlap between the plunger rod 820 and the outer electrode 810 is twice their area of overlap at stopper position 830a. Because the measured capacitance between electrodes 810 and 820 is proportional to their overlapping area, the measured capacitance increases in a linear manner as the plunger rod 820 and stopper 830 move through the container. Thus, the measured capacitance indicates the amount of drug remaining in the container.

FIG. 9 is a side view of a syringe with a tapered outer electrode and a stopper inner electrode, according to an example useful for understanding the invention. In this example, an electrode 910 is arranged on an outer wall of the container. The electrode 910 is tapered, with its width varying along the length of the container. A stopper 930 forms a second electrode. For example, the stopper 930 may contain a conductive material or conductive coating. The position of the stopper 930 is controlled by a plunger rod 920, which may or may not be conductive, and may or may not form a portion of the inner electrode. The plunger rod 920 electrically couples the stopper 930, or the electrode portion of the stopper 930, to a voltage source and measurement circuit, e.g., the measurement circuit 450 of FIG. 4. In this example, the wall of the syringe is the dielectric between the two electrodes 910 and 920; the drug or other substance held by the syringe is not between the electrodes 910 and 920 and capacitance through the drug is not measured.

Two example stopper positions 930a and 930b are shown in FIG. 9. As the plunger rod 920 and stopper 930 move within the container, the amount of overlap between the stopper 930 and outer electrode 910 decreases, due to the varying width of the electrode 910. In particular, the overlapping area between the stopper 930a and the outer electrode 910 is represented as shaded area 940a, and the overlapping area between the stopper 930b and the outer electrode 910 is represented as shaded area 940b, which is smaller than shaded area 940a. Because the measured capacitance between electrodes 910 and 930 is proportional to their overlapping area, the measured capacitance decreases as the plunger rod 920 and stopper 930 move through the container. Thus, the measured capacitance indicates the position of the stopper 930 in the container, which correlates to the amount of drug remaining in the container.

FIG. 10 is a side view of a syringe with two tapered outer electrodes and a stopper inner electrode, according to an example useful for understanding the invention. This embodiment includes an outer electrode 1010 similar to the outer electrode 910, a plunger rod 1020 similar to the plunger rod 920, and a stopper 1030 similar to the stopper 930. As in the embodiment of FIG. 9, the position of the stopper 1030 is controlled by the plunger rod 1020, which may or may not be conductive, and may or may not form a portion of the inner electrode.

This embodiment further includes a second outer electrode 1040 is on the opposite side of the container from the first outer electrode 1010. In this example, the second outer electrode 1040 is tapered in an opposite direction from the first outer electrode 1010. The voltage source and measurement circuit may be configured to take capacitance measurements between different pairs of electrodes, e.g., one measurement between the inner electrode 1030 and the first outer electrode 1010, and another measurement between the inner electrode 1030 and the second outer electrode 1040. Taking capacitance measurements between the inner electrode 1030 and two outer electrodes 1010 and 1040 improves utility of the sensor. For example, at the stopper position 1030a, the measurement to the first outer electrode 1010 may be more accurate, because the overlap between the stopper 1030 and the first outer electrode 1010 is larger than the overlap between the stopper 1030 and the second outer electrode 1040, whereas at the stopper position 1030b, the measurement to the second outer electrode 1040 may be more accurate.

It should be understood that the electrode configurations shown in FIGs. 5-10 are illustrative, and other electrode configurations may be used. For example, in the examples shown in FIGs. 8 and 9, a second outer electrode matching the shape and arrangement of the first outer electrode 810 or 910 may be arranged on the opposite side of the syringe from the first outer electrode 810 or 910. A measurement system may obtain capacitance measurements from alternating pairs of electrodes, e.g., alternating between the inner electrode and one outer electrode and between the inner electrode and the other outer electrode; alternating between an inner electrode and an outer electrode and between the two outer electrodes; or alternating between all three electrode pairs.

### Electrode Shields

The capacitive sensors described above apply a voltage difference to a pair of electrodes to generate an electric field across the electrodes, and measure the capacitance across the pair of electrodes. Outside disturbances to the electric field, such as other medical devices, cell phones, other devices, the patient, medical staff, etc. can affect the accuracy of the capacitance measurement. The capacitive sensors described above can incorporate conforming electrode shields to protect the capacitive sensor from such outside disturbances to the electric field.

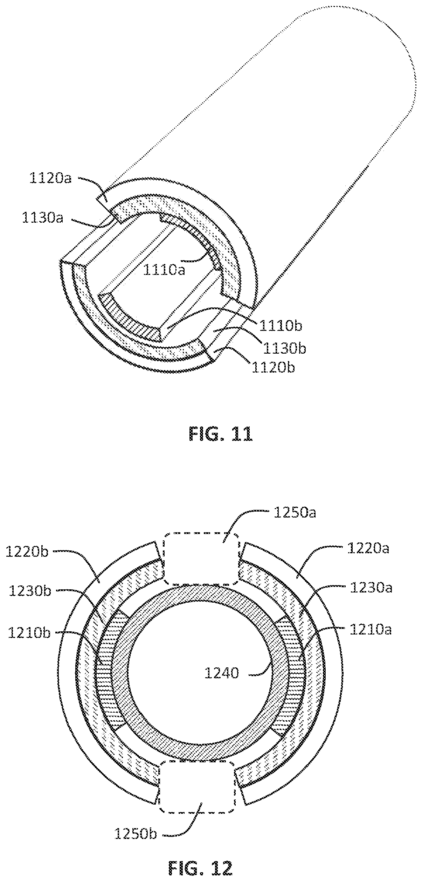

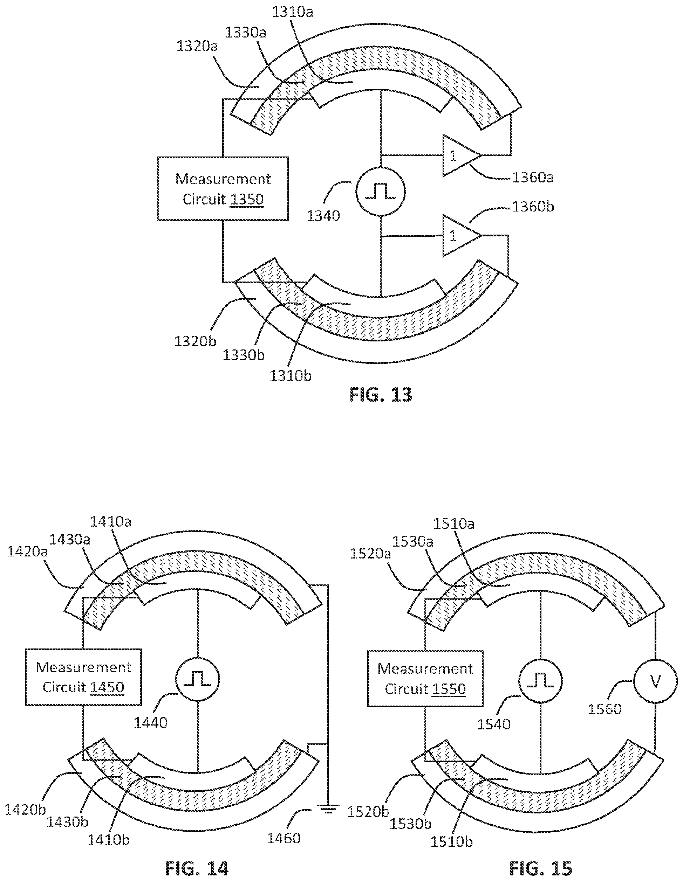

FIG. 11 is a perspective view of a pair of shielded electrodes, according to an example useful for understanding the invention. Two electrodes 1110a and 1110b correspond to any of the electrodes shown in the prior figures, e.g., any of electrodes 150, 250, 330, or 430. While the electrodes 1110 are shown as being rectangular, the electrodes 1110a and 1110b may have any of the shapes described with respect to FIGs. 5-10. One electrode shield 1120a partially encloses the electrode 1110a, and another electrode shield 1120b partially enclose the other electrodes 1110b. The electrode shields 1120 may be composed of a conductive substance, such as a metal. A time-varying voltage may be applied across the electrode shields 1120a and 1120b, or the electrode shields may be set to a fixed potential, as described with respect to FIGs. 13-15. The electrode shield 1120a is separated from the electrode 1110a by an insulating layer 1130a, and the electrode shield 1120b is separated from the electrode 1110b by an insulating layer 1130b. The insulating layers 1130 are formed from a nonconductive material.

FIG. 12 is a cross-section of a container with the shielded electrodes shown in FIG. 11, according to an example useful for understanding the invention. FIG. 12 shows cross-sections of the electrodes 1210a and 1210b, which are similar to the electrodes 1110a and 1110b; electrode shields 1220a and 1220b, which are similar to the electrode shields 1120a and 1120b; and insulating layers 1230a and 1230b, which are similar to the insulating layers 1130a and 1130b. A container wall 1240 is arranged within the electrodes 1210, insulating layers 1230, and electrode shields 1220. Two viewing windows 1250a and 1250b provide visual access to the container between gaps in the electrodes 1210, insulating layers 1230, and electrode shields 1220. The viewing windows 1250 separate the pairs of electrodes 1210a and 1210b, insulating layers 1230a and 1230b, and electrode shields 1220a and 1220b.

The electrode shields 1220 and insulating layers 1230 extend across a larger arc than the electrodes 1210. Extending the electrode shields 1220 beyond the arc of the electrodes 1210 increases the amount of shielding provided, which leads to a more accurate capacitance measurement. However, extending the electrode shields 1220 and insulating layers 1230 reduces the size of the viewing windows 1250. In other embodiments, a single, cylindrical electrode shield and a single insulating layer may wrap fully around the electrodes 1210a and 1210b and the wall 1240. This may provide superior shielding to the arrangement shown in FIG. 12, however, it eliminates visual access to the container.

While the examples shown in FIGs. 11 and 12, and the examples shown in FIGs. 13-19 and described below, show a sensor system with two outer electrodes on the wall of a container or syringe, similar shielding arrangements with electrode shields separated by viewing windows may be used in containers with one inner electrode and one outer electrode. For example, in the arrangement of FIG. 12, the second electrode 1210b may be omitted, and the insulating layer 1230b is adjacent to the wall 1240. The insulating layer 1230b may be thicker than the insulating layer 1230a, so that the electrode shields 1220a and 1220b are the same distance from the wall 1240. It should be understood that any of the arrangements in FIGs. 11-19 can be similarly adapted for use in containers with one outer electrode and an inner electrode.

If the plunger rod is used as an inner electrode, the electrode shields may extend the length of the outer electrode plus the length of the plunger rod when the plunger rod is positioned at the syringe's fullest position. For example, for the syringe shown in FIG. 2, the electrode shields may extend from the left side of the syringe by the full length of the plunger rod 260.

FIGs. 13-15 show example electrical configurations of the measurement electrodes and electrode shields. FIG. 13 is a cross-section showing shielded electrodes in which the electrode shields are driven by a buffer driver, according to an example useful for understanding the invention. FIG. 13 includes a pair of electrodes 1310a and 1310b, a pair of electrode shields 1320a and 1320b, and a pair of insulating layers 1330a and 1330b. The electrodes 1310 are coupled to a voltage source 1340, which may be similar to the voltage source 340 described with respect to FIG. 3. The electrodes 1310 are also coupled to a measurement circuit 1350, which may be similar to the measurement circuit 350 described with respect to FIG. 3.

The voltage source 1340 has two terminals, one coupled to a first electrode 1310a and another coupled to a second electrode 1310b. Each of the terminals of the voltage source 1340 are coupled to a respective buffer circuit 1360a and 1360b. The buffer circuits 1360 are represented as amplifiers with a gain of one. The buffer circuits 1360a and 1360b are further coupled to the electrode shields 1320a and 1320b, respectively, to apply the same voltage difference to the electrode shields 1320 that is applied across the electrodes 1310. In other words, in the absence of interfering signals, the first electrode shield 1320a has the same voltage as the first electrode 1310a, and the second electrode shield 1320b has the same voltage as the second electrode 1310b. The buffer circuits 1360 replicate the voltage signal from the voltage source 1340 while protecting the voltage signal applied to the electrodes 1310a and 1310b. If a stray electric field affects the voltage levels of the electrode shields 1320, the buffer circuits 1360 prevent the voltage levels from affecting the voltage difference applied to the electrodes 1310. In other embodiments, the sensor system includes one buffer circuit, e.g., if one electrode (e.g., electrode 1310a) and one electrode shield (e.g., electrode shield 1320a) are grounded, or if the buffer circuit can buffer a differential voltage signal.

FIG. 14 is a cross-section showing shielded electrodes in which the electrode shields are grounded, according to an example useful for understanding the invention. FIG. 14 includes a pair of electrodes 1410a and 1410b, a pair of electrode shields 1420a and 1420b, and a pair of insulating layers 1430a and 1430b. The electrodes 1410 are coupled to a voltage source 1440, which may be similar to the voltage source 340 described with respect to FIG. 3. The electrodes 1410 are also coupled to a measurement circuit 1450, which may be similar to the measurement circuit 350 described with respect to FIG. 3. In this example, the electrode shields 1420a and 1420b are coupled to a ground 1460. For example, the ground 1460 may be a ground of the measurement circuit 1450 and/or voltage source 1440. Grounding the electrode shields 1420 may provide less effective shielding than the buffer-driven shields 1320 shown in FIG. 13. However, connecting the electrode shields 1420 to a ground 1460 allows for a simpler circuit with fewer parts, which may reduce size and expense. Furthermore, the circuit of FIG. 14 may be more robust, e.g., it may have a longer life or lower chance of failure than the circuit of FIG. 13.

In certain use cases, such as where doses are applied over long period of time, or minor dosing or measurement errors are not unsafe for the patient, the shielding provided by the electrode shields 1420 may be sufficient. Furthermore, for applications that are not time sensitive, a sensor system may be programmed to take multiple measurements to reduce the likelihood of measurement error caused by an errant electric field. For example, the sensor system may be programmed to capture multiple capacitance measurements at different times (e.g., two measurements 1 minute apart) while holding the stopper stationary and, if the measurements differ by at least a threshold amount, repeat the capacitance measurements at periodic intervals until a stable measurement is obtained.

FIG. 15 is a cross-section showing shielded electrodes in which the electrode shields are driven by a different voltage source from the electrodes, according to an example useful for understanding the invention. FIG. 15 includes a pair of electrodes 1510a and 1510b, a pair of electrode shields 1520a and 1520b, and a pair of insulating layers 1530a and 1530b. The electrodes 1510 are coupled to a first voltage source 1540, which may be similar to the voltage source 340 described with respect to FIG. 3. The electrodes 1510 are also coupled to a measurement circuit 1550, which may be similar to the measurement circuit 350 described with respect to FIG. 3. In this example, the electrode shields 1520a and 1520b are coupled to a second voltage source 1560. The second voltage source 1560 may apply a fixed or variable voltage difference across the electrode shields 1520. For example, the second voltage source 1560 may apply the same waveform as the first voltage source 1540, at the same amplitude or at a different amplitude. As another example, the second voltage source 1560 may apply a fixed potential to one or both electrode shields 1520a and 1520b, e.g., a supply voltage of the measurement circuit 1550 and/or voltage source 1540. In some embodiments, the second voltage source 1560 applies a voltage potential to one of the electrode shields (e.g., electrode shield 1520a), and the other electrode shield (e.g., electrode shield 1520b) is grounded.

### Guarded Electrode Shields

In certain implementations, the electrode shields described above may not provide sufficient shielding. For example, certain equipment, such as magnetic resonance imaging (MRI) machines, or certain environments, such as helicopters, have higher amounts of electric field disturbance, and sensor systems used in such environments can benefit from enhanced shielding. Furthermore, more accurate measurements may be desirable for certain use cases. For example, if the capacitive sensor is measuring a chemotherapy drug or other toxic drug where an excessive dose can be dangerous for the patient, a high assurance of accuracy is desired. To provide additional protection from outside electric fields, additional layers of electrode shields may be incorporated. For example, an inner, active electrode shield may be guarded by an outer electrode shield.

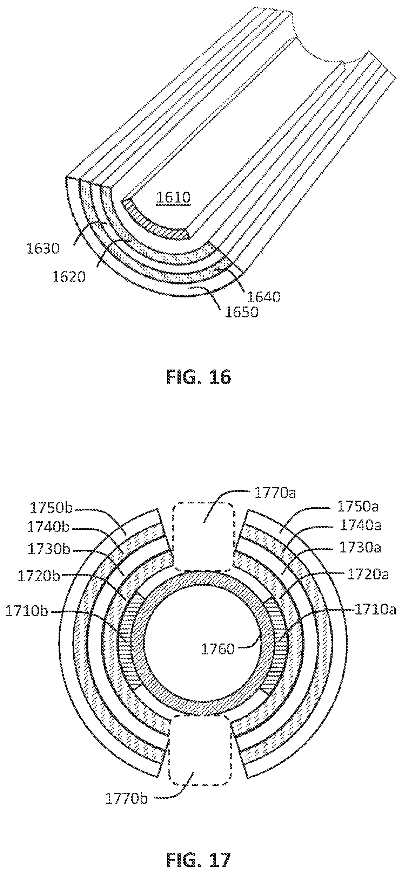

FIG. 16 is a perspective view of a sensor electrode with inner and outer electrode shields, according to some embodiments of the present disclosure. An electrode 1610 corresponds to any of the electrodes shown in FIGs. 1-10, e.g., any of electrodes 150, 250, 330, or 430. While the electrode 1610 is shown as being rectangular, the electrode 1610 may have any of the shapes described with respect to FIGs. 5-10. The electrode 1610 is partially enclosed by a first insulating layer 1620, an inner electrode shield 1630, a second insulating layer 1640, and an outer electrode shield 1650, which are jointly referred to as a guarded electrode shield. The electrode shields 1630 and 1650 may be composed of a conductive substance, such as a metal. The insulating layers 1620 and 1640 are formed from a nonconductive material.

FIG. 17 is a cross-section of a container with the inner and outer electrode shields, according to some embodiments of the present disclosure. FIG. 17 shows cross-sections of the electrodes 1710a and 1710b, which are examples of the electrode 1610; insulating layers 1720a and 1720b, which are examples of the insulating layer 1620; inner electrode shields 1730a and 1730b, which are examples of the electrode shield 1630; insulating layers 1740a and 1740b, which are examples of the insulating layer 1640; and outer electrode shields 1750a and 1750b, which are examples of the outer electrode shield 1650. A container wall 1660 is arranged within the electrodes 1710 and guarded electrode shields 1720-1750. Two viewing windows 1770a and 1770b provide visual access to the container between gaps in the electrodes 1710 and guarded electrode shields 1720-1750. The viewing windows 1770 separate the pair of electrodes 1710 and the pair of guarded electrode shields.

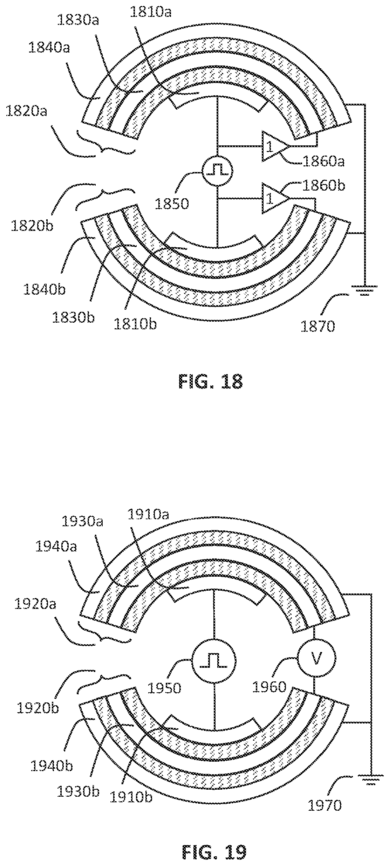

FIG. 18 is a cross-section showing inner and outer electrode shields in which the inner shields are driven by a buffer driver and the outer shields are grounded, according to some embodiments of the present disclosure. FIG. 18 includes a pair of electrodes 1810a and 1810b and a pair of guarded electrode shields 1820a and 1820b. Each guarded electrode shield 1820 includes an inner electrode shield 1830 and an outer electrode shield 1840. The electrodes 1810 are coupled to a voltage source 1850, which may be similar to the voltage source 340 described with respect to FIG. 3. The electrodes 1810 are also coupled to a measurement circuit (not shown) as described above.

The voltage source 1850 has two terminals, one coupled to a first electrode 1810a and another coupled to a second electrode 1810b. Each of the terminals of the voltage source 1850 are coupled to a respective buffer circuit 1860a and 1860b, which are similar to the buffer circuits 1360a and 1360b described with respect to FIG. 13. The buffer circuits 1860a and 1860b are further coupled to the inner electrode shields 1830a and 1830b, respectively, to apply the same voltage difference to the inner electrode shields 1830 that is applied across the electrodes 1810. The outer electrode shields 1840 are coupled to a ground 1870. For example, the ground 1870 may be a ground of the measurement circuit and/or voltage source 1850.

As described with respect to FIG. 13, in the presence of an outside electric field, electrode shields 1320a and 1320b with a buffered voltage absorb much of the stray charge, so the electric field between the two electrodes 1310 used to obtain the capacitance measurement is minimally affected. When stationary external electric fields (or no external fields) are present in the environment of the sensor, replicating the voltages of the electrodes 1310 at the electrode shields 1320 results in no current flow between the electrodes 1310 and the electrode shields 1320. However, a variable external charge (e.g., a smartphone moving nearer to a drug delivery device implementing the capacitance sensor) that is absorbed by the electrode shields 1320 causes the voltages of the electrode shields 1320 to temporarily be different from the electrodes 1310, causing current to flow between the electrode shields 1320 and the electrodes 1310. This current can impact accuracy of the impedance measurement between the electrodes 1310 until the circuit can restore the correct voltages on the electrode shields 1320.

The guarded electrode shields 1820 shown in FIG. 18 prevent current flow between the inner electrode shields 1830 and the electrodes 1810. The outer electrode shields 1840 protect the inner electrode shields 1830, which minimizes stray current between the inner shields 1830 and the electrodes 1810. If a stray charge reaches an inner electrode shield (e.g., inner electrode shield 1830a), a current flows from the inner electrode shield 1830a to the grounded outer electrode shield 1840a, rather than from the inner electrode shield 1830a to the electrode 1810a. This minimizes stray currents on the electrodes 1810, which maintains the integrity of the capacitance measurement across the electrodes 1810.

FIG. 19 is a cross-section showing inner and outer electrodes in which the inner shields are driven by a different voltage source from the electrodes and the outer shields are grounded, according to some embodiments of the present disclosure. FIG. 19 includes a pair of electrodes 1910a and 1910b and a pair of guarded electrode shields 1920a and 1920b. Each guarded electrode shield 1920 includes an inner electrode shield 1930 and an outer electrode shield 1940. The electrodes 1910 are coupled to a voltage source 1950, which may be similar to the voltage source 340 described with respect to FIG. 3. The electrodes 1910 are also coupled to a measurement circuit (not shown) as described above.

In this example, the inner electrode shields 1930a and 1930b are coupled to a second voltage source 1960. The second voltage source 1960 may apply a fixed or variable voltage difference across the inner electrode shields 1930. For example, the second voltage source 1960 may apply the same waveform as the first voltage source 1950, at the same amplitude or at a different amplitude. As another example, the second voltage source 1960 may apply a fixed potential to one or both inner electrode shields 1930a and 1930b. In some embodiments, the second voltage source 1960 applies a voltage potential to one of the inner electrode shields (e.g., inner electrode shield 1930a), and the other inner electrode shield (e.g., inner electrode shield 1930b) is grounded.

The outer electrode shields 1940 are coupled to a ground 1970. For example, the ground 1970 may be a ground of the measurement circuit and/or voltage source 1950. In some embodiments, the second voltage source 1960 is a ground, e.g., both the inner electrode shields 1930a and 1930b and the outer electrode shields 1940a and 1940b are coupled to the ground 1970.

While the example containers shown in FIGs. 1-19 are cylindrical, it should be understood that the capacitive sensors and electrode shields described herein may have different shapes. For example, the container may have an oval, square, or rectangular cross-section. In some embodiments, the cross-section of the container may vary along its length, e.g., the container may be tapered towards one end. The shape of the electrodes and the shape of the electrode shield may be adapted to conform to the shape of the container. Furthermore, while the examples described above generally show a container with a stopper, such as a syringe, the capacitive sensors described herein may be used for vials or other containers that do not include a stopper, e.g., to measure the volume of a drug filled into a vial or removed from a vial.

While the examples described above generally discuss dispensing a drug or other material from a container and measuring the remaining drug as it is delivered, the capacitive sensors described above may be used in a similar matter to measure a drug or other material being loaded into a syringe or other container, e.g., to measure a volume of drug filled into a syringe.

### Drug Delivery System

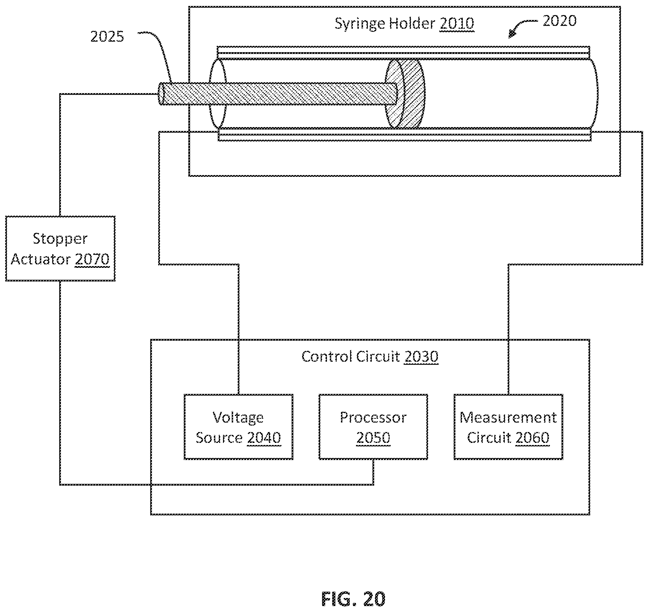

Any of the capacitive sensors described with respect to FIGs. 1-19 can be used in a drug delivery system to automatically dispense a drug from a container, e.g., a syringe. FIG. 20 is a block diagram showing a drug delivery system according to an example useful for understanding the invention. The drug delivery system includes a syringe holder 2010, a control circuit 2030, and a stopper actuator 2070. The syringe holder 2010 holds a syringe 2020 controlled by a plunger rod 2025. The control circuit 2030 includes a voltage source 2040, a processor 2050, and a measurement circuit 2060. In alternative configurations, different, fewer, and/or additional components may be included in the drug delivery system from those shown in FIG. 20. Furthermore, the functionality described in conjunction with one or more of the components shown in FIG. 20 may be distributed among the components in a different manner than described.

The syringe holder 2010 is configured to hold a syringe containing a drug. The syringe holder 2010, the syringe 2020, and/or the plunger rod 2025 provide a capacitive sensor and conforming electrode shields, e.g., any of the capacitive sensors described with respect to FIGs. 1-10, and any of the conforming electrode shields described with respect to FIGs. 11-19. In some embodiments, the syringe holder 2010 is configured to hold one or more standard syringes, e.g., a syringe of a particular standard size and shape. In some embodiments, the syringe holder 2010 includes a capacitive sensor and conforming electrode shields, which fit around the syringe 2020 when the syringe 2020 is inserted into the syringe holder 2010. For example, the syringe holder 2010 can be designed to accept existing FDA-approved syringes. In other embodiments, the syringe 2020 (and in some embodiments, the plunger rod 2025) includes the capacitive sensor and conforming electrode shields, and the syringe holder 2010 includes electrical contacts for coupling the capacitive sensor and electrode shields to the control circuit 2030. In some embodiments, the syringe 2020 and, optionally, the plunger rod 2025 includes the capacitive sensor, and the syringe holder 2010 includes the conforming electrode shields along with electrical contacts to the electrodes of the capacitive sensor.

As used herein, a drug is any substance suitable for delivery via a syringe, such as a biopharmaceutical, synthesized pharmaceutical, blood or blood product, saline, etc. The drug has a different permittivity than air, so the change in measured capacitance corresponds to a change in the relative volumes of drug and air between the electrodes. Typically, drugs have a higher permittivity than air, so the measured capacitance decreases as the drug is pushed out of the syringe and the volume of air between the electrodes increases. In the example in which one of the electrodes is the plunger rod, as the plunger rod moves through the syringe, the size of the capacitor (i.e., the amount of overlap between the electrode on the wall of the syringe and the conductive plunger) increases, which increases the measured capacitance.

The voltage source 2040 connects to the capacitive sensor to apply a voltage to the electrodes. The voltage source 2040 may be the voltage source 340, voltage source 455, or any of the voltage sources shown in FIGs. 13-15 or FIGs. 18-19. In some embodiments, the control circuit 2030 includes a second voltage source for the electrode shields, e.g., as shown in FIGs. 15 and 19.

The stopper actuator 2070 can be coupled to the plunger rod 2025 to physically control the position of the plunger rod 2025 and, by extension, the position of the stopper in the syringe 2020. The processor 2050 sends instructions to the stopper actuator 2070 to move the plunger rod 2025. For example, the processor 2050 may send an instruction to the stopper actuator 2070 to apply a particular force to the plunger rod 2025, or to apply force to the plunger rod 2025 for a particular duration.

The processor 2050 further determines the amount of drug remaining in the syringe. In particular, the measurement circuit 2060 connects to the electrodes of the capacitive sensor and determines a capacitance measurement, e.g., based on a measured charge. The measurement circuit 2060 provides the capacitance measurement to the processor 2050, and the processor 2050 determines, based on the measured capacitance, the amount of drug remaining in the syringe. The processor 2050 may have calibration data indicating, for a given drug, the amount of remaining drug that corresponds to different capacitance levels. Alternatively, the processor 2050 may store a formula for converting the measured capacitance to an amount of remaining drug.

In some embodiments, the drug delivery system controls the movement of the stopper based on the amount of the drug that has been delivered or that remains in the syringe. For example, the processor 2050 receives an instruction to deliver a specific volume of the drug to a patient. The processor 2050 instructs the stopper actuator 2070 to move by a specific amount, e.g., a first portion of the instructed volume. The processor 2050 obtains periodic capacitance measurements from the measurement circuit 2060 and calculates the volume of drug that has been delivered to the patient. If the volume to be delivered has not been delivered, the processor 2050 sends further instructions to the stopper actuator 2070 to move the plunger rod 2025. The processor 2050 continues this process and determines to stop instructing the stopper actuator 2070 to push the drug out of the syringe after the instructed volume has been delivered.

In some embodiments, the processor 2050 may output the amount of remaining drug in the syringe 2020. For example, the drug delivery system may have a display screen for displaying the amount of remaining drug and/or the amount of drug that has been delivered. The drug delivery system may be in wireless or wired communication with one or more other devices, and transmit the amount of remaining or delivered drug to the other device(s).

### Further Applications of Capacitive Sensor

The capacitive sensors described herein can be used for additional applications relating to drug delivery and drug monitoring. For example, a capacitive sensor can determine a type of drug within a syringe, or confirm if a drug in a syringe is an expected drug. In particular, different drugs may have different relative permittivities. To identify a particular drug, a syringe or other container (e.g., a vial) containing the drug is placed in a capacitive sensing system having the capacitive sensor described above. The capacitive sensing system can determine the permittivity of the drug in the container based on the measured capacitance. The permittivity may be compared to a database of permittivities for different drugs to identify the drug. Alternatively, if a particular drug is expected, the permittivity is compared to a permittivity for the expected drug to confirm if the expected drug is present in the container.

As another example, the permittivity of a drug can be measured and used to confirm whether a drug has fouled. In this example, if a drug has been improperly stored (e.g., not refrigerated), has been kept for too long, has been contaminated, or has otherwise changed composition, the permittivity of the drug may change. The drug may be tested before use by measuring its permittivity and confirming that the permittivity indicates that the drug has not fouled.

As another example, the permittivity of a drug can be measured and used to determine the concentration of the drug. A given drug at different concentrations may have different relative permittivities. The drug may be tested before use by measuring its permittivity and confirming that the permittivity indicates that the drug is at the correct concentration.

### Other Implementation Notes, Variations, and Applications

It is to be understood that not necessarily all objects or advantages may be achieved in accordance with any particular embodiment described herein. Thus, for example, those skilled in the art will recognize that certain embodiments may be configured to operate in a manner that achieves or optimizes one advantage or group of advantages as taught herein without necessarily achieving other objects or advantages as may be taught or suggested herein.

In one example embodiment, any number of electrical circuits of the figures may be implemented on a board of an associated electronic device. The board can be a general circuit board that can hold various components of the internal electronic system of the electronic device and, further, provide connectors for other peripherals. More specifically, the board can provide the electrical connections by which the other components of the system can communicate electrically. Any suitable processors (inclusive of digital signal processors, microprocessors, supporting chipsets, etc.), computer-readable non-transitory memory elements, etc. can be suitably coupled to the board based on particular configuration needs, processing demands, computer designs, etc. Other components such as external storage, additional sensors, controllers for audio/video display, and peripheral devices may be attached to the board as plug-in cards, via cables, or integrated into the board itself. In various embodiments, the functionalities described herein may be implemented in emulation form as software or firmware running within one or more configurable (e.g., programmable) elements arranged in a structure that supports these functions. The software or firmware providing the emulation may be provided on non-transitory computer-readable storage medium comprising instructions to allow a processor to carry out those functionalities.

## Claims

1. A sensor for measuring contents of a container (100), the sensor comprising:
a voltage source (1850) to generate a first voltage;
a pair of electrodes (1810a, 1810b) coupled to the voltage source, the pair of electrodes to apply an electric field extending through at least a portion of an interior of the container,
wherein a first electrode (1810a) of the pair of electrodes extends along a wall (140) of the container, and
wherein: a second electrode (1810b) of the pair of electrodes extends along the wall of the container on an opposite side of the container to the first electrode, or a second electrode (230, 260) of the pair of electrodes is within the container;
a measurement circuit configured to measure a capacitance across the pair of electrodes;
an inner shield (1830a, 1830b) partially enclosing the pair of electrodes, the inner shield having a second voltage; and
an outer shield (1840a, 1840b) partially enclosing the inner shield, the outer shield having a third voltage (1870).

2. The sensor of claim 1, the inner shield comprising a first portion (1830a) and a second portion (1830b), the first portion partially enclosing a first of the pair of electrodes (1810a), the second portion partially enclosing a second of the pair of electrodes (1810b), and the second voltage is a voltage difference between the first portion and the second portion.

3. The sensor of claim 2, the voltage difference between the first portion and the second portion of the inner shield equal to a voltage difference applied by the voltage source to the pair of electrodes.

4. The sensor of claim 2, the outer shield comprising a first portion (1840a) and a second portion (1840b), the first portion of the outer shield partially enclosing the first portion of the inner shield, the second portion of the outer shield partially enclosing the second portion of the inner shield, and the third voltage is a fixed voltage applied to both the first portion of the outer shield and the second portion of the outer shield.

5. The sensor of any of claims 1-4, further comprising an insulating layer (1740a, 1740b) between the inner shield and the outer shield.

6. The sensor of any of claims 1-5, wherein the second electrode of the pair of electrodes extends along the wall of the container on the opposite side of the container to the first electrode.

7. The sensor of claim 6, wherein the first electrode and second electrode are separated by a pair of viewing windows (1770a, 1770b) to provide visual access to contents of the container.

8. The sensor of claim 6, wherein the first electrode and the second electrode are rectangular.

9. The sensor of claim 6, wherein the first electrode is tapered towards a first end of the container, and the second electrode is tapered towards the first end of the container.

10. The sensor of claim 1, wherein the second electrode of the pair of electrodes is within the container.

11. The sensor of claim 10, wherein the container is a syringe, and the second electrode of the pair of electrodes comprises a plunger rod (260) of the syringe.

12. The sensor of claim 10, wherein the container is a syringe, and the second electrode of the pair of electrodes comprises a stopper (230) of the syringe.

13. The sensor of claim 10, wherein the first electrode is rectangular.

14. The sensor of claim 10, wherein the first electrode is tapered toward an end of the container.

15. The sensor of claim 10, further comprising a third electrode (430b) extending along the wall of the container on an opposite side of the container to the first electrode.

## Patentansprüche

1. Sensor zum Messen des Inhalts eines Behälters (100), wobei der Sensor Folgendes umfasst:
eine Spannungsquelle (1850) zum Erzeugen einer ersten Spannung;
ein Elektrodenpaar (1810a, 1810b), das mit der Spannungsquelle gekoppelt ist, wobei das Elektrodenpaar ein elektrisches Feld anlegt, das sich durch mindestens einen Abschnitt eines Innenraums des Behälters erstreckt,
wobei sich eine erste Elektrode (1810a) des Elektrodenpaars entlang einer Wand (140) des Behälters erstreckt, und
wobei: eine zweite Elektrode (1810b) des Elektrodenpaars sich entlang der Wand des Behälters auf einer gegenüberliegenden Seite des Behälters zu der ersten Elektrode erstreckt, oder eine zweite Elektrode (230, 260) des Elektrodenpaars sich innerhalb des Behälters befindet;
eine Messschaltung zum Messen einer Kapazität über das Elektrodenpaar ausgebildet ist;
eine innere Abschirmung (1830a, 1830b) das Elektrodenpaar teilweise umschließt, wobei die innere Abschirmung eine zweite Spannung aufweist; und
eine äußere Abschirmung (1840a, 1840b) die innere Abschirmung teilweise umschließt, wobei die äußere Abschirmung eine dritte Spannung (1870) aufweist.

2. Sensor nach Anspruch 1, wobei die innere Abschirmung einen ersten Abschnitt (1830a) und einen zweiten Abschnitt (1830b) umfasst, wobei der erste Abschnitt ein erstes des Elektrodenpaars (1810a) teilweise umschließt, wobei der zweite Abschnitt ein zweites des Elektrodenpaars (1810b) teilweise umschließt, und die zweite Spannung ein Spannungsunterschied zwischen dem ersten Abschnitt und dem zweiten Abschnitt ist.

3. Sensor nach Anspruch 2, wobei der Spannungsunterschied zwischen dem ersten Abschnitt und dem zweiten Abschnitt der inneren Abschirmung einem von der Spannungsquelle an das Elektrodenpaar angelegten Spannungsunterschied entspricht.

4. Sensor nach Anspruch 2, wobei die äußere Abschirmung einen ersten Abschnitt (1840a) und einen zweiten Abschnitt (1840b) umfasst, wobei der erste Abschnitt der äußeren Abschirmung den ersten Abschnitt der inneren Abschirmung teilweise umschließt, der zweite Abschnitt der äußeren Abschirmung den zweiten Abschnitt der inneren Abschirmung teilweise umschließt, und die dritte Spannung eine feste Spannung ist, die sowohl auf den ersten Abschnitt der äußeren Abschirmung als auch auf den zweiten Abschnitt der äußeren Abschirmung angewendet wird.

5. Sensor nach einem der Ansprüche 1 bis 4, weiter umfassend eine Isolierschicht (1740a, 1740b) zwischen der inneren und der äußeren Abschirmung.

6. Sensor nach einem der Ansprüche 1 bis 5, wobei sich die zweite Elektrode des Elektrodenpaars entlang der Wand des Behälters auf der gegenüberliegenden Seite des Behälters zu der ersten Elektrode erstreckt.

7. Sensor nach Anspruch 6, wobei die erste Elektrode und die zweite Elektrode durch ein Paar Sichtfenster (1770a, 1770b) getrennt sind, um einen visuellen Zugang zu dem Inhalt des Behälters bereitzustellen.

8. Sensor nach Anspruch 6, wobei die erste Elektrode und die zweite Elektrode rechteckig sind.

9. Sensor nach Anspruch 6, wobei die erste Elektrode zu einem ersten Ende des Behälters spitz zuläuft und die zweite Elektrode zu dem ersten Ende des Behälters spitz zuläuft.

10. Sensor nach Anspruch 1, wobei sich die zweite Elektrode des Elektrodenpaars innerhalb des Behälters befindet.

11. Sensor nach Anspruch 10, wobei der Behälter eine Spritze ist und die zweite Elektrode des Elektrodenpaars eine Kolbenstange (260) der Spritze umfasst.

12. Sensor nach Anspruch 10, wobei der Behälter eine Spritze ist und die zweite Elektrode des Elektrodenpaars einen Stopper (230) der Spritze umfasst.

13. Sensor nach Anspruch 10, wobei die erste Elektrode rechteckig ist.

14. Sensor nach Anspruch 10, wobei die erste Elektrode zu einem Ende des Behälters spitz zuläuft.

15. Sensor nach Anspruch 10, weiter umfassend eine dritte Elektrode (430b), die sich entlang der Wand des Behälters auf einer gegenüberliegenden Seite des Behälters zu der ersten Elektrode erstreckt.

## Revendications

1. Capteur de mesure du contenu d'un récipient (100), le capteur comprenant :
une source de tension (1850) pour générer une première tension ;
une paire d'électrodes (1810a, 1810b) couplée à la source de tension, la paire d'électrodes servant à appliquer un champ électrique s'étendant à travers au moins une portion d'un intérieur du récipient,
dans lequel une première électrode (1810a) de la paire d'électrodes s'étend le long d'une paroi (140) du récipient, et
dans lequel : une deuxième électrode (1810b) de la paire d'électrodes s'étend le long de la paroi du récipient sur un côté opposé du récipient par rapport à la première électrode, ou une deuxième électrode (230, 260) de la paire d'électrodes est à l'intérieur du récipient ;
un circuit de mesure configuré pour mesurer une capacité à travers la paire d'électrodes ;
un blindage interne (1830a, 1830b) entourant partiellement la paire d'électrodes, le blindage interne présentant une deuxième tension ; et
un blindage externe (1840a, 1840b) entourant partiellement le blindage interne, le blindage externe présentant une troisième tension (1870).

2. Capteur selon la revendication 1, le blindage interne comprenant une première portion (1830a) et une deuxième portion (1830b), la première portion entourant partiellement une première électrode de la paire d'électrodes (1810a), la deuxième portion entourant partiellement une deuxième électrode de la paire d'électrodes (1810b), et la deuxième tension est une différence de tension entre la première portion et la deuxième portion.

3. Capteur selon la revendication 2, la différence de tension entre la première portion et la deuxième portion du blindage interne est égale à une différence de tension appliquée par la source de tension à la paire d'électrodes.

4. Capteur selon la revendication 2, le blindage externe comprenant une première portion (1840a) et une deuxième portion (1840b), la première portion du blindage externe entourant partiellement la première portion du blindage interne, la deuxième portion du blindage externe entourant partiellement la deuxième portion du blindage interne, et la troisième tension est une tension fixe appliquée à la fois à la première portion du blindage externe et à la deuxième portion du blindage externe.

5. Capteur selon l'une quelconque des revendications 1 à 4, comprenant en outre une couche isolante (1740a, 1740b) entre le blindage interne et le blindage externe.

6. Capteur selon l'une quelconque des revendications 1 à 5, dans lequel la deuxième électrode de la paire d'électrodes s'étend le long de la paroi du récipient sur le côté opposé du récipient par rapport à la première électrode.

7. Capteur selon la revendication 6, dans lequel la première électrode et la deuxième électrode sont séparées par une paire de fenêtres de visualisation (1770a, 1770b) pour fournir un accès visuel au contenu du récipient.

8. Capteur selon la revendication 6, dans lequel la première électrode et la deuxième électrode sont rectangulaires.

9. Capteur selon la revendication 6, dans lequel la première électrode se rétrécit en direction d'une première extrémité du récipient, et la deuxième électrode se rétrécit en direction de la première extrémité du récipient.

10. Capteur selon la revendication 1, dans lequel la deuxième électrode de la paire d'électrodes est à l'intérieur du récipient.

11. Capteur selon la revendication 10, dans lequel le récipient est une seringue, et la deuxième électrode de la paire d'électrodes comprend une tige de piston (260) de la seringue.

12. Capteur selon la revendication 10, dans lequel le récipient est une seringue, et la deuxième électrode de la paire d'électrodes comprend un bouchon (230) de la seringue.

13. Capteur selon la revendication 10, dans lequel la première électrode est rectangulaire.

14. Capteur selon la revendication 10, dans lequel la première électrode se rétrécit en direction d'une extrémité du récipient.

15. Capteur selon la revendication 10, comprenant en outre une troisième électrode (430b) s'étendant le long de la paroi du récipient sur un côté opposé du récipient par rapport à la première électrode.
